(19) Europäisches Patentamt
European Patent Office
Office européen des brevets

(11) Publication number: 0 006 845
B1

(12) # EUROPEAN PATENT SPECIFICATION

(45) Date of publication of patent specification: 03.02.82

(21) Application number: 78100344.7

(22) Date of filing: 10.07.78

(51) Int. Cl.³: A 61 K 31/60,
A 61 K 31/40 //(A61K31/60,
31/40), (A61K31/60, 31/40,
31/165)

(54) **Pharmaceutical composition comprising a 5-aroyl-1-C1-5 alkyl-pyrrole-2-acetic acid compound and acetaminophen or acetylsalicylic acid.**

(43) Date of publication of application:
23.01.80 Bulletin 80/2

(45) Publication of the grant of the European patent:
03.02.82 Bulletin 82/5

(84) Designated Contracting States:
BE CH DE FR GB LU NL SE

(56) References cited:
US - A - 3 752 826

(73) Proprietor: McNeilab, Inc.
Camp Hill Road
Fort Washington Pennsylvania (US)

(72) Inventor: Wong, Stewart
527 Summit Avenue
Fort Washington Pennsylvania (US)

(74) Representative:
Vossius.Vossius.Tauchner.Heunemann.Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 006 845 B1

Pharmaceutical composition comprising a 5-aroyl-1-$C_{1-5}$ alkyl pyrrole-2-acetic acid compound and acetaminophen or acetylsalicyclic acid

The invention relates to a pharmaceuticall composition comprising a 5-aroyl-1-loweralkyl-pyrrole-2-acetic acid compound and acetaminophen or acetylsalicylic acid.

Within the past few years, a new class of non-hormonal anti-inflammatory agents namely, certain 5-aroyl-1-loweralkyl-pyrrole-2-acetic acid derivatives, have been reported. Due to their anti-inflammatory activity, such derivatives are indicated for the inflammation and pain associated with arthritic diseases, e.g., rheumatoid arthritis, osteoarthritis and the like. It has now been found that such derivatives, when combined with two well-known analgesics, acetaminophen acetylsalicyclic acid, have greater efficacy in the suppression of inflammation and arthritic degenerations than when administered alone.

The 5-aroyl-1-loweralkyl-pyrrole-2-acetic acid derivatives to be employed in this invention are those encompassed within the following structural formula

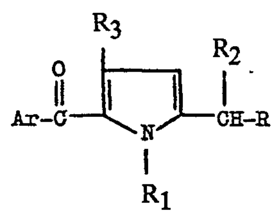

(I)

wherein:

R is CN, COOH,
COO (loweralkyl), $CONH_2$, CONH(loweralkyl) or CON(loweralkyl)$_2$;
$R_1$ is loweralkyl;
$R_2$ is hydrogen or loweralkyl;
$R_3$ is hydrogen,
lower alkyl, chloro or bromo, provided that when said $R_3$ is chloro or bromo, then said R is COOH; and
Ar is phenyl,
trifluoromethylphenyl, methylthiophenyl or phenyl substituted with one to three substitutents being loweralkyl, loweralkoxy
and halo; with the proviso that when $R_3$ is hydrogen, then Ar is other than loweralkylphenyl
and the non-toxic, therapeutically acceptable salts of the foregoing acids, i.e., when R is COOH, such as are obtained from appropriate organic or inorganic bases.

As used herein, "loweralkyl" and "loweralkoxy" means straight or branch chained saturated hydrocarbons having from 1 to 5 carbon atoms, such as for example, methyl, ethyl, propyl, isopropyl, butyl, and pentyl, and, respectively, the corresponding alkoxys such as, methoxy, ethoxy, propoxy, and isopropoxy; and "halo" represents chloro, fluoro, bromo and iodo.

The anti-inflammatory compounds of formula (I) are described in U.S. Patent No 3,752,826.

The preferred compounds of formula (I) for the novel combinations of this invention are those embraced by the formula:

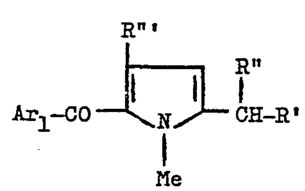

(II)

wherein:

R′ is COOH or COO(loweralkyl);
R″ is hydrogen or methyl;
R‴ is methyl, ethyl,
chloro or bromo, provided that when said R‴ is chloro or bromo, then said R′ is COOH; and $Ar_1$ is phenyl,
methylthiophenyl, loweralkylphenyl, loweralkoxyphenyl or halophenyl;
and the alkali metal salts of the foregoing acids, i.e., when R′ is COOH.

The more preferred compounds of formula (II) for the novel combinations of this invention are those wherein the substituted phenyls within the term "Ar", are para-substituted. One of the most preferred compounds is 1,4-dimethyl-5-*p*-chlorobenzoyl-pyrrole-2-acetic acid, generically known as

"ZOMEPIRAC" [see J. Med. Chem., *14*, 646 (1971); J. Med. Chem., *16*, 172 (1973); and J. Pharmcol. Exptl. Therm., *185*, 127 (1973)].

When one component of a combination is known to possess a certain pharmacological property and such property is increased many-fold, or known side-effects are concurrently eliminated or reduced, when said component is combined with one or more other drugs, then the net effect of the combination is commonly referred to as "potentiation".

It has now been found that a potentiation of the anti-inflammatory activity possessed by the above-described 5-aroyl-1-loweralkyl-pyrrole-2-acetic acid derivatives is produced by a combination with acetylsalicylic acid or acetaminophen in specified proportions. It has also been found that the latter two drugs also potentiate the anti-arthritic activity of the former derivatives as evidenced by an increased suppression of bone-degenerative changes. Such unique and surprising potentiations are not merely due to the additive effects of the individual components but, rather, are made possible solely and entirely by the action of the combination itself.

It is evident, therefore, that the novel combinations of this invention would find useful applications in alleviating the inflammation, and particularly inflammation, pain and bone degenerative changes associated with arthritic diseases, e.g., rheumatoid arthritis, osteoarthritis and the the like.

The efficacy of the novel combination of this invention in inhibiting inflammation and osteogenic degeneration is particularly seen in the adjuvant arthritis test. Although a number of acute antiphlogistic tests have been devised for the study of inflammation and although inflammation is a common feature of these tests and arthritis, every type of inflammation does not lead to articular (joint) tissue dammage that is associated with arthritis. The adjuvant arthritis test, a test in which adjuvant arthritis which results both in inflammation and osteogenic changes is induced by *Mycobacterium butyricum* and in which the effect of the test compounds on each effect can be determined, is considered to be most useful for evaluation of compounds which may be suitable for the treatment of rheumatoid arthritis and other arthritic diseases. The test procedure, in which the effect on inflammation is determined by paw volume changes and the effect on osteogenic changes is determined by microscopic observation, is described by Wong et al, in J. Pharmacol, Exp. Ther. *185*, 127—138, 1973, and is employed in the present determination of the potentiation of the antiarthritic properties of 5-aroyl-1-loweralkyl-pyrrole-2-acetic acid derivatives of acetylsalicylic acid or acetaminophen.

Briefly, in this procedure, adjuvant arthritis is induced in female Wistar, Lewis rats (Charles River Breeding Laboratories, Inc., Wilmington, Mass.) weighing 160—190 gm by a single subcutaneous injection of 0.75 mg *Mycobacterium butyricum* (Difco) into the left hind paw. The non-injected hind paw remains a normal size for the first seven days. Swelling begins to appear in the non-injected paw of the first rats on Day 8. Using the paw volume determination technique (a technique in which rat paws are dipped in mercury to the hairline and the volume of displacement determined by a modified Van Arman mercury displacement method as described in the aforementioned paper of Wong et al), the time progress curve of volume changes in the non-injected (contralateral) paw is followed. Since it has been found that whenever an animal shows edema $\geq$ 0.25 ml, further increase in the contralateral paw size always follows with swelling developing rapidly during the next two to three days, the 0.25 ml edema is used as criterion of arthritis onset. The mean onset time for 100 rats is found to be 11.6 days with a standard deviation of 1.9 days and the frequency of onset time values distributed normally between Days 8 and 19. Thus, determinations of antiarthritic activity are made in the period Day 11 to Day 28.

It has been found that the progress of adjuvant arthritis can be divided into four phases: Phase I (generally Days 0—10) is the incubation period, Phase II (generally Days 11—8), the time of rapid development of swelling, Phase III (generally Days 19—25), the period of established adjuvant arthritis and Phase IV (generally after 25 Days), the phase of osteogenic changes.

For determination of anti-inflammatory properties two different evaluations are made: (a) the evaluation of paw volume during the Phase II and Phase III periods and, (b) the evaluation of osteogenic (bone) degeneration during the Phase IV period.

For evaluation of paw volume, adjuvant arthritic rats with early but significant signs of arthritis in the contralateral paw are selected on Day 11 and randomly assigned to the various groups of 10 animals in each group. All test animals are dosed daily (per os) with the test compound, preferably as sodium salt, or in saline for 17 days (Days 11 to 27 inclusive). Control animals are dosed with an equivalent volume of saline. The paw volumes of the non-injected paws are determined initially on Day 11 and again on Days 15, 18, 22, 25, and 28. The paw volumes are then compared with normal paw volumes of adjuvant arthritic rats previously determined from a reference curve relating normal paw volume to body weight. The volume of each individual rat which is greater than the normal volume is utilized in the analysis of the data. Values for drug treated animals are expressed as a percent inhibition of the paw volume change relative to the mean value for saline controls. Data is evaluated statistically and $ED_{50}$ values with 95 percent confidence limits are calculated. The method for statistical analysis is described in detail in the aforementioned paper of Wong et al. The term "$ED_{50}$" refers to the dose of drug required to produce 50 percent antagonism of the paw volume changes observed in the saline treated adjuvant arthritis controls. Suppression of paw volume changes reflects antiinflammatory and antiarthritic activity of a test drug.

For evaluation of bone degenerations, normal and adjuvant arthritic rats are sacrificed under $CO_2$, after the paw volume measurements have been completed on Day 28. The hind legs are removed above the knees and skinned and the soft tissues dissected away with care to avoid injury to bone and articular structures. The leg bones are then immersed in 2 percent potassium hydroxide solution for approximately four to five days until the remaining soft tissues becomes properly macerated and/or transparent, and the bones are fully visible. The leg bones are stained with Alizarin Red (0.01 percent in 2 percent KOH) for eight hours, and then processed through increasing concentrations of glycerol in water (20, 40, 60, 80 and 100 percent) for purposes of clearing the tissues for microscopic observation. The distal end of the tibiae, the tarsals, metatarsals, phalanges, and sesamoids are evaluated under a Stereozoom Microscope (a special type of dissecting microscope, product of Bausch and Lomb).

Osteogenic changes for each bone (in 31 bone categories) in the non-injected hind paw are graded on a scale of from 0 to 10 (increasing numerical value corresponding to increasing severity of bone degeneration). A total bone degeneration score is obtained (maximum score/paw = 310 points) and then expressed as a percent score. The mean percent score (95 percent confidence limits) for saline control groups from 26 experiments (184 animals) is found to be 53.4 (50.9—55.9). Percent score values for drug treated animals are expressed as a percent inhibition relative to the mean for the saline control group. Data is evaluated statistically and $ED_{50}$ values with 95 percent confidence limits are calculated.

Employing the above described procedures, potentiation of antiarthritic activity of 5-aroyl-1-loweralkyl-pyrrole-2-acetic acid derivatives by acetaminophen and acetylsalicylic acid are determined. The application of the above-described procedure is particularly illustrated with 1,4-dimethyl-5-p-chlorobenzoyl-pyrrole-2-acetic acid and 4-chloro-5-p-chlorobenzoyl-1-methyl-pyrrole-2-acetic acid. It is to be understood that the compounds illustrated are not for purposes of limiting the invention thereto but only to show the useful properties of compounds within the scope of Formula I. (In the experiments hereinafter described, the 5-aroyl-1-loweralkyl compounds are employed as sodium salts but are calculated and expressed as free acids.)

In paw volume studies, the $ED_{50}$ for acetaminophen on the 28th day, and its 95 percent confidence limits (C.L.), are 576 (504—698) mg/kg/day. The minimum effective does (MED) for acetaminophen is found to be 320 mg/kg/day. In bone degeneration studies the $ED_{50}$ for acetaminophen and its 95 percent C.L. are 1673 (1563—2008) mg/kg/day with a regression coefficient and 95 percent C.L. 32.3 (31.0—33.6).

In paw volume studies for acetylsalicylic acid the $ED_{50}$ and its 95 percent C.L. are found to be 322 (299—545) mg/kg/day. Acetylsalicylic acid alone shows a 16.4 $\pm$ 11.4 percent inhibition at 25 mg/kg/day and a 28.3 $\pm$ 8.2 percent inhibition at 100 mg/kg/day. Both of these responses are not significantly different from the saline control (DSD = 30.8 percent). In bone degeneration studies the $ED_{50}$ for acetylsalicylic acid and its 95 percent C.L. for suppression of osteogenic changes in adjuvant arthritis has been shown to be 382 (367—464) mg/kg/day with a regression coefficient $\pm$ 95 percent C.L. 33.4 (32.1—34.6).

MED is the dose of drug which will produce a statistically significant difference ($p < 0.05$) from saline treated controls according to Dunnett's procedure described in Wong et al. For these experiments the mean ($\pm$ Standard Error) for Dunnett's significant different (DSD) is 23.8 $\pm$ 3.2 percent inhibition.

*Potentiation of Anti-Inflammatory Activity of Compounds of Formula I*

Using the paw volume technique previously described, the effect of acetaminophen or acetylsalicylic acid on 5-aroyl-1-loweralkyl-pyrrole-2-acetic acid compound of formula I are determined.

Table I shows the results obtained when 1,4-dimethyl-5-p-chlorobenzoyl-pyrrole-2-acetic acid alone, acetaminophen alon and the combinations of the two agents are administered in various doses. The $ED_{50}$ values for each case are indicated.

TABLE I

Percent Inhibition of Paw Volume ± S.E. (Day 28)

| Dose of 1,4-Dimethyl-5-p-chlorobenzoyl-pyrrole-2-acetic acid (mg/kg/day) | Dose of Acetaminophen 0 | 100 | 200 | 400 |
|---|---|---|---|---|
| 0 | | 2.7 ± 17.4 | 6.6 ± 11.4 | 31.6 ± 6.1 |
| 0.11 | 27.4 ± 10.1 | 35.3 ± 8.9 | 39.1 ± 12.3 | 61.2 ± 3.2 |
| 0.33 | 45.0 ± 6.0 | 48.3 ± 7.0 | 57.8 ± 2.8 | 58.4 ± 4.3 |
| 1.0 | 68.0 ± 7.3 | 74.9 ± 6.2 | 70.0 ± 3.8 | 84.5 ± 5.3 |
| $ED_{50}$ (mg/kg) | 0.40 | 0.36 | 0.21 | 0.04 |

The data is evaluated by analysis of variance (as set forth in Wong et al, supra) for a 4 × 5 factorial complete block design, with days representing the block. Comparison of means are carried out using Dunnett's procedure at the 5 percent special protection level.

Table II shows the results obtained when 1,4-dimethyl-5-p-chlorobenzoyl-pyrrole-2-acetic acid alone, acetylsalicylic acid alone and the combinations of the two agents are administered in various doses.

TABLE II

Percent Inhibition of Paw Volume ± S.E. (Day 28)

| Dose of 1,4-Dimethyl-5-p-chlorobenzoyl-pyrrole-2-acetic acid (mg/kg/day) | Dose of Acetylsalicyclic Acid (mg/kg/day) 0 | 75 | 150 | 300 |
|---|---|---|---|---|
| 0 | | 4.1 ± 14.4 | 33.2 ± 7.6 | 28.9 ± 9.4 |
| 0.125 | 17.5 ± 7.5 | 17.3 ± 3.4 | 23.6 ± 9.0 | 51.0 ± 5.2 |
| 0.5 | 43.8 ± 11.8 | 45.6 ± 12.3 | 48.6 ± 9.4 | 48.9 ± 8.3 |
| 2.0 | 68.2 ± 5.3 | 67.9 ± 6.3 | 73.5 ± 5.5 | 64.3 ± 5.0 |
| $ED_{50}$ (mg/kg) | 0.728 | 0.712 | 0.541 | 0.283 |

Table III shows the results obtained when 4-chloro-5-p-chlorobenzoyl-1-methyl-pyrrole-2-acetic acid alone, acetaminophen alone and the combination of the two agents are administered in various doses.

TABLE III

Percent Inhibition ± S.E. (Day 28)

| Dose of 4-Chloro-5-p-chlorobenzoyl-1-methyl-pyrrole-2-acetic acid (mg/kg/day) | Dose of Acetaminophen (mg/kg/day) 0 | 50 | 200 | 800 |
|---|---|---|---|---|
| 0 | | 7.6 ± 6.1 | 29.0 ± 12.8 | 75.3 ± 3.9 |
| 0.02 | 18.6 ± 11.7 | 41.2 ± 11.7 | 48.7 ± 5.4 | 80.5 ± 3.6 |
| 0.06 | 61.3 ± 5.3 | 58.1 ± 9.1 | 75.0 ± 2.3 | 91.1 ±7.2 |
| 0.18 | 78.9 ± 8.7 | 84.0 ± 5.7 | 87.6 ± 6.1 | 88.2 ± 5.8 |
| $ED_{50}$ (mg/kg) | 0.054 | 0.032 | 0.019 | <0.001 |

The foregoing results illustrate the potentiation of the anti-inflammatory and antiarthritic properties of 5-aroyl-1-loweralkyl-pyrrole-2-acetic acid derivatives by acetaminophen and acetylsalicylic acid. The properties are utilized in the compositions of the present invention.

The invention also relates to the use of a 5-aroyl-1-loweralkyl-pyrrole-2-acetic derivative of Formula I or its pharmaceutically acceptable salt as primary active agent together with acetaminophen or acetylsalicylic acid as potentiating agent for preparing a pharmaceutical composition for inhibiting inflammations and osteogenic degenerations.

The operative ranges of the combination of 5-aroyl-1-loweralkyl-pyrrole-2-acetic acid derivative and acetaminophen or acetylsalicylic acid depends to some extent on whether the potentiating agent is acetaminophen or acetylsalicylic acid. Generally when acetaminophen is the potentiating agent, from 50 to 800 mg/kg of acetaminophen is employed together with from 0.001 to 100 mg/kg of the 5-aroyl-1-loweralkyl-pyrrole-2-acetic acid compound, and preferably from 100 to 400 mg/kg of acetaminophen with from 0.1 to 50 mg/kg of the 5-aroyl-1-loweralkyl-pyrrole-2-acetic acid compound. When acetylsalicylic acid is the potentiating agent, from about 50 to 300 mg/kg of acetylsalicylic acid is employed with from 0.001 to 100 mg/kg of the 5-aroyl-1-loweralkyl-pyrrole-2-acetic acid compound, and preferably from 100 to 150 mg/kg of acetylsalicylic acid with from 0.01 to 50 mg/kg of the 5-aroyl-1-loweralkyl-pyrrole-2-acetic acid compound.

The outstanding properties are most effectively utilized by use of the novel pharmaceutical compositions of the present invention. To prepare the pharmaceutical compositions of this invention, a 5-aroyl-1-loweralkyl-pyrrole-2-acetic acid compound and a potentiating agent acetylsalicylic acid or acetaminophen are intimately admixed with a pharmaceutically acceptable carrier suitable for oral administration. By the expression "5-aroyl-1-loweralkyl-pyrrole-2-acetic acid compound" as employed above and in the claims is meant not only the compound defined by Formulas I and II, but the pharmaceutically acceptable salts thereof. In preparing the compositions in oral dosage form, any of the usual pharmaceutical media may be employed, such as for example, water, glycols, oils, alcohols and the like for oral liquid preparations such as suspensions, elixirs and solutions; or solid carriers such as starches, sugars, kaolin, lubricants, binders disintegrating agents and the like for powders, capsules and tablets. Because of their ease in administration, tablets and capsules represent the most advantageous oral dosage form, in which case solid pharmaceutical carriers are employed. It is especially advantageous to formulate the aforementioned pharmaceutical compositions in dosage unit form for ease of administration and uniformity of dosage. The term "dosage unit form" as used in the specification and claims herein refers to physically discrete units suitable as unitary dosages, each unit containing a predetermined quantity of active ingredient calculated to produce the desired therapeutic effect in association with the required pharamaceutical carrier. Examples of such dosage unit forms are tablets, capsules, pills, powder packets, wafers, teaspoonfuls, tablespoonfuls and the like, and segregated multiples thereof. A dosage unit generally will contain from 0.1 to 1000 mg of a 5-aroyl-1-loweralkyl-pyrrole-2-acetic acid compound as primary active ingredients together with from 150 to 1000 mg of acetylsalicylic acid or from 150 to 2000 mg of acetaminophen. The preferred dosage unit is from 0.2 to 500 mg of a 5-aroyl-1-loweralkyl-pyrrole-2-acetic acid compound together with from 325 to 500 mg of acetylsalicylic acid or from 325 to 1000 mg of acetaminophen.

The following examples are given to illustrate the novel compositions and are not to be construed as limiting the invention in spirit or in scope.

Example I

100 hard gelatin capsules, each containing 4.6 milligrams of 5-p-chlorobenzoyl-1,4-dimethyl-pyrrole-2-acetic acid as primary active ingredient and 325 milligrams of acetaminophen as potentiating agent are prepared from the following formulation:

| | Grams |
|---|---|
| 5-p-Chlorobenzoyl-1,4-dimethyl-pyrrole-2-acetic acid | 4.6 |
| Acetaminophen | 325 |
| Starch | 250 |
| Lactose | 750 |
| Talc | 250 |
| Calcium stearate | 10 |

A uniform mixture of the ingredients is prepared by blending and filled into two-piece hard gelatin capsules. The capsules are suitable to be used for providing satisfactory inhibition of inflammation upon administration to subjects with articular inflammation.

Example II

Gelatin capsules are prepared as described in Example I except that in the formulations, 5.2 grams of potassium 5-p-chlorobenzoyl-1,4-dimethyl-pyrrole-2-acetate is employed as the primary active ingredient and 325 grams of acetylsalicylic acid is substituted as the potentiating agent, thus providing capsules each containing 5.2 mg of potassium 5-p-chlorobenzoyl-1,4-dimethyl-pyrrole-2-acetate and 325 milligrams of acetylsalicylic acid.

**Claims**

1. A pharmaceutical composition comprising an effective inflammation and osteogenic degeneration inhibiting amount of

(1) from 0.1 to 1000 mg of a 5-aroyl-1-$C_{1-5}$alkyl-pyrrole-2-acetic acid compound having the formula I

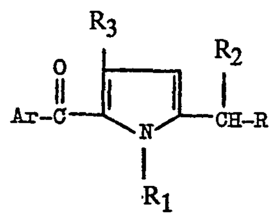

(I)

wherein:

R is CN, COOH, COO($C_{1-5}$alkyl), $CONH_2$, CONH($C_{1-5}$alkyl) or CON($C_{1-5}$alkyl)$_2$;

$R_1$ is $C_{1-5}$ alkyl;

$R_2$ is hydrogen or $C_{1-5}$ alkyl;

$R_3$ is hydrogen, $C_{1-5}$alkyl, chloro or bromo, provided that when said $R_3$ is chloro or bromo, then said R is COOH; and

Ar is phenyl, trifluoromethylphenyl, methylthiophenyl or phenyl substituted with one to three substituents being $C_{1-5}$alkyl, $C_{1-5}$alkoxy or chloro, fluoro, bromo or iodo, with the proviso that when $R_3$ is hydrogen, then Ar is other than $C_{1-5}$alkylphenyl;

or a non-toxic therapeutically acceptable salt of the foregoing acids, namely, acids when R is COOH as primary active agent; and

from 150 to 2000 mg of acetaminophen or from 150 to 1000 mg of acetylsalicylic acid as potentiating agent

wherein said primary and potentiating agents are in admixture with a pharmaceutically acceptable carrier.

2. A composition according to Claim 1 in which the compound of Formula I is 5-p-chlorobenzoyl-1,4-dimethyl-pyrrole-2-acetic acid or a non-toxic, therapeutically acceptable salt thereof and the potentiating agent is acetaminophen.

3. A composition according to Claim 1 in which the compound of Formula I is 5-p-chlorobenzoyl-1,4-dimethyl-pyrrole-2-acetic acid or a non-toxic, therapeutically acceptable salt thereof and the potentiating agent is acetylsalicylic acid.

4. A composition according to Claim 1 in which the dosage unit form is a tablet.

5. A composition according to Claim 1 in which the dosage unit form is a capsule.

6. A pharmaceutical composition suitable for inhibiting inflammation and osteogenic degeneration in dosage unit form comprising per dosage unit

(1) from 0.1 to 1000 mg of a 5-aroyl-1-loweralkyl-pyrrole-2-acetic acid compound having the Formula II

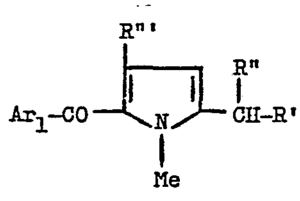

(II)

wherein:

R′ is COOH, COO($C_{1-5}$alkyl) or COO(alkali metal);

R″ is hydrogen or methyl;

R‴ is methyl, ethyl, chloro or bromo, provided that when R‴ is chloro or bromo, then said R′ is COOH; and

$Ar_1$ is phenyl, methylthiophenyl, $C_{1-5}$alkylphenyl, $C_{1-5}$alkoxyphenyl or phenyl substituted by chloro, fluoro, bromo or iodo

as primary active agent; and

(2) from 150 to 1000 mg of acetylsalicylic acid or from 150 to 2000 mg of acetaminophen, as potentiating agent
wherein said primary and potentiating agents are in admixture with a pharmaceutically acceptable carrier.

7. A composition according to Claim 6 comprising dosage unit
(1) from 0.2 to 500 mg of a compound of Formula II as primary active agent; and
(2) from 325 to 500 mg of acetylsalicylic acid or from 325 to 1000 mg of acetaminophen as potentiating agent wherein said primary and potentiating agents are in admixture with a pharmaceutically acceptable carrier.

8. Use of the compounds (1) and (2) as set forth in claim 1 for the preparation of a pharmaceutical composition according to claim 1 which inhibits inflammation and osteogenic degeneration.

**Patentansprüche**

1. Arzneimittel enthaltend eine zur Hemmung von Entzündungen und Knochendegenerationen wirksame Menge von
(1) von 0,1 bis 1000 mg einer 5-Aroyl-1-$C_{1-5}$-alkyl-pyrrol-2-essigsäure der Formel I

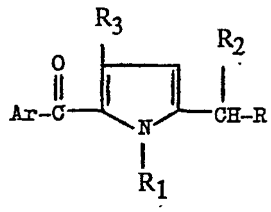

in der
R CN,COOH, COO($C_{1-5}$-alkyl), $CONH_2$, CONH($C_{1-5}$-alkyl) oder CON($C_{1-5}$-alkyl)$_2$ ist,
$R_1$ $C_{1-5}$-alkyl ist,
$R_2$ Wasserstoff oder $C_{1-5}$- Alkyi
$R_3$ Wasserstoff, $C_{1-5}$-alkyl, Chlor oder Brom ist, mit der Maßgabe, daß R COOH bedeutet, wenn $R_3$ Chlor oder Brom ist, und
Ar Phenyl, Trifluormethylphenyl oder Methylthiophenyl oder durch $C_{1-5}$-alkyl, $C_{1-5}$-alkoxy oder Chlor, Fluor, Brom oder Jod 1 bis 3-fach substituiertes Phenyl ist, mit der Maßgabe, daß Ar nicht $C_{1-5}$-alkylphenyl ist, wenn $R_3$ Wasserstoff bedeutet.
oder ein nicht toxisches, therapheutisch verträgliches Salz der genannten Säuren, nämlich Säuren, wenn R COOH darstellt,
als Hauptwirkstoff;
(2) von 150 bis 2000 mg Acetaminophen oder von 150 bis 1000 mg Acetylsalicylsäure als Potenzierungsmittel,
wobei der Huptwirkstoff und das Potenzierungsmittel mit einem pharmazeutisch verträglichen Trägerstoff vermischt sind.

2. Arzneimittel nach Anspruch 1, in dem die Verbindung der Formel I 5-p-Chlorbenzoyl-1,4-dimethyl-pyrrol-2-essigsäure oder ein nicht toxisches, therapeutisch verträgliches Salz davon und das Potenzierungsmittel Acetaminophen ist.

3. Arzneimittel nach Anspruch 1, in dem die Verbindung der Formel I 5-p-Chlorbenzoyl-1,4-dimethyl-pyrrol-2-essigsäure oder ein nicht toxisches, therapeutisch verträgliches Salz davon und das Potenzierungsmittel Acetylsalicylsäure ist.

4. Arzneimittel nach Anspruch 1, dadurch gekennzeichnet, daß die Dosiseinheit eine Tablette ist.

5. Arzneimittel nach Anspruch 1, dadurch gekennzeichnet, daß die Dosiseinheit eine Kapsel ist.

6. Arzneimittel zur Hemmung von Entzündungen und Knochendegenerationen in Dosiseinheitsform, enthaltend pro Dosiseinheit
(1) von 0,1 bis 1000 mg einer 5-Aroyl-1niederalkylpyrrol-2-essigsäure der allgemeinen Formel II

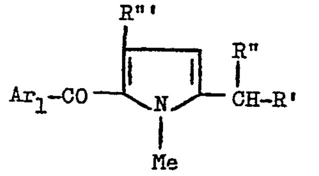

(II)

in der
R' COOH, COO($C_{1-5}$-alkyl) oder COO(Alkalimetall) ist
R'' Wasserstoff oder Methyl ist

R''' Methy, Äthyl, Chlor oder Brom ist, mit der Maßgabe, daß R' COOH darstellt, wenn R''' Chlor oder Brom ist, und

$Ar_1$ Phenyl, Methylthiophenyl, $C_{1-5}$-alkylphenyl, $C_{1-5}$-alkoxyphenyl oder durch Chlor, Fluor, Brom oder Jod substituiertes Phenyl ist,

als Haputwirkstoff, und

(2) von 150 bis 1000 mg Acetylsalicylsäure oder von 150 bis 2000 mg Acetaminophen als Potenzierungsmittel

wobei der Hauptwirkstoff und das Potenzierungsmittel mit einem pharmazeutisch verträglichen Trägerstoff vermischt sind.

7. Arzneimittel nach Anspruch 6, enthaltend pro Dosiseinheit.

(1) von 0,2 bis 500 mg einer Verbindung der Formel II als Hauptwirkstoff und

(2) von 325 bis 500 mg Acetylsalicylsäure oder von 325 bis 1000 mg Acetaminophen als Potenzierungsmittel, wobei der Hauptwirkstoff und das Potenzierungsmittel mit einem pharmazeutisch verträglichen Trägerstoff vermischt sind.

8. Verwendung der Verbindungen (1) und (2) gemäß Anspruch 1 zur Herstellung eines Arzneimittels gemäß Anspruch 1, das Entzündung und Knochendegeneration hemmt.

**Revendications**

1. Composition pharmaceutique qui comprend en quantité efficace pour inhiber l'inflammation et la dégénérescence ostéogène.:

(1) 0,1 à 1.000 mg d'un dérivé d'un acide 5-aroyle-1-alcoyle en $C_{1-5}$-pyrrole-2-acétique de formule:

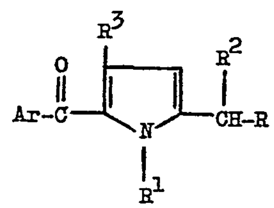

(I)

où

R représente un radical CN, COOH, COO(alcoyle en $C_{1-5}$), $CONH_2$, CONH(alcoyle en $C_{1-5}$) ou CON(alcoyle en $C_{1-5})_2$;

$R^1$ représente un radical alcoyle en $C_{1-5}$;

$R^2$ représente un atome d'hydrogène ou un radicai alcoyle en $C_{1-5}$;

$R^3$ représente un atome d'hydrogène, de chlore ou de brome ou un radical alcoyle en $C_{1-5}$, étant entendu que lorsque $R^3$ représente un atome de chlore ou de brome, R représente un radical COOH, et

Ar représente un radical phényle, trifluorométhylphényle, méthylthiophényle ou phényle substitué portant un à trois substituants qui sont des radicaux alcoyle en $C_{1-5}$ ou alcoxy en $C_{1-5}$ ou des atomes de chlore, de fluor, de brome ou d'iode, étant entendu que lorsque $R^3$ représente un atome d'hydrogène, Ar ne représente pas un radical alcoyle en $C_{1-5}$-phényle; ou d'un sel non toxique pharmaceutiquement acceptable des acides ci-dessus, à savoir les acides où R représente un radical COOH, comme principe actif principal, et

(2) 150 à 2.000 mg d'acétaminophène ou 150 à 1.000 mg d'acide acétylsalicylique, comme agent de potantialisation,

le principe actif et l'agent de potentialisation étant en mélange avec un excipient pharmaceutiquement acceptable.

2. Composition suivant la revendication 1, dans laquelle le composé de formule I est l'acide 5-p-chlorobenzoyl-1,4-diméthylpyrrole-2-acétique ou un de ses sels non toxiques pharmaceutiquement acceptables et l'agent de potentialisation est l'acétaminophène.

3. Composition suivant la revendication 1, dans laquelle le composé de formule I est l'acide 5-p-chlorobenzoyl-1,4-diméthylpyrrole-2-acétique ou un de ses sels non toxiques pharmaceutiquement acceptables et l'agent de potentialisation est l'acide acétylsalicylique.

4. Composition suivant la revendication 1, dont la forme dosée unitaire est un comprimé.

5. Composition suivant la revendication 1, dont la forme dosée unitaire est une capsule.

6. Composition pharmaceutique propre á inhiber l'inflammation et la dégénérescence ostéogène, en formes dosées unitaires, qui comprend, par dose unitaire,

(1) 0,1 à 1.000 mg d'un dérivé d'un acide 5-aroyle-1-alcoyle inférieur-pyrrole-2-acétique de formule:

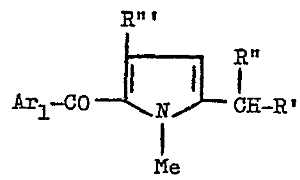

(II)

où

R' représente un radical COOH, COO(alcoyle en $C_{1-5}$) ou COO(métal alcalin);

R" représente un atome d'hydrogène ou un radical méthyle;

R''' représente un radical méthyle ou éthyle ou un atome de chlore ou de brome, étant entendu que lorsque R''' représente un atome de chlore ou de brome, R' représente un radical COOH, et

$Ar_1$ représente un radical phényle, méthylthiophényle, alcoyle en $C_{1-5}$-phényle, alcoxy en $C_{1-5}$-phényle ou phényle substitué par un des atomes de chlore, de fluor, de brome ou d'iode comme principe actif principal, et

(2) 150 à 1.000 mg d'acide acétylsalicylique ou 150 à 2.000 mg d'acétaminophène comme agent de potentialisation,

le principe actif et l'agent de potentialisation étant en mélange avec un excipient pharmaceutiquement acceptable.

7. Composition suivant la revendication 6 qui comprend, par dose unitaire:

(1) 0,2 à 500 mg d'un composé de formule II, comme principe actif principal, et

(2) 325 à 500 mg d'acide acétylsalicylique ou 325 à 1.000 mg d'acétaminophène, comme agent de potentialisation,

le principe actif et l'agent de potentialisation étant en mélange avec un excipient pharmaceutiquement acceptable.

8. Utilisation des composés (1) et (2) tels que définis dans la revendication 1 pour la préparation d'une composition pharmaceutique suivant la revendication 1 qui inhibe l'inflammation et la dégénérescence ostéogène.